# EUROPEAN PATENT APPLICATION

(11) **EP 3 851 115 A1**
(43) Date of publication of application: **21.07.2021**
(21) Application number: 20382019.6
(22) Date of filing: 15.01.2020
(51) Int. Cl.: A61K 35/745, A61K 35/747, A23L 33/135, A61K 9/48, A61P 3/00

(54) **PROBIOTIC COMPOSITION FOR ITS USE AS AN ANTIOXIDANT**

(71) Applicant: Biopolis, S.L., 46980 Paterna, Valencia (ES)
(72) Inventor: CHENOLL CUADROS, Maria Empar, 46185 La Pobla de Vallbona (Valencia) (ES); MARTORELL GUEROLA, Patricia, 46220 Picassent (Valencia) (ES); GENOVÉS MARTÍNEZ, Salvador, 46960 Aldaia (Valencia) (ES); RAMÓN VIDAL, Daniel, 46183 La Eliana (Valencia) (ES); LÓPEZ ROMÁN, Francisco Javier, 30506 Molina de Segura (Murcia) (ES); ÁVILA GANDÍA, Vicente, 30107 Murcia (ES); CÁNOVAS GARCÍA, Fernando, 30009 Murcia (ES)
(74) Representative: Pons

(57) **Abstract**

This invention refers to a probiotic composition consisting of the bacteria *Lactobacillus rhamnosus, Lactobacillus casei* and *Bifidobacterium longum,* preferably of the strains *L. rhamnosus* CECT8361, *L. casei* CECT9104 and *B. longum* CECT7347, and its use as an antioxidant. This composition is particularly useful in the treatment and / or prevention of damage, at the molecular level, caused by oxidative stress, preferably during high intensity physical exercise.

## Description

The present invention falls within the field of Medicine, preferably sports medicine, and probiotic foods or nutritional supplements that exert an antioxidant effect. In particular, the invention is included within the probiotic food or pharmaceutical compositions aimed at reducing oxidative stress, preferably that caused by intense physical exercise.

### STATE OF THE ART

Reactive oxygen species (ROS), such as superoxide anion (O₂-), hydrogen peroxide (H₂O₂) and hydroxyl radical (HO•), are radical and non-radical oxygen species caused by the partial reduction of oxygen. Cellular ROS are generated endogenously in the mitochondrial oxidative phosphorylation process, or may arise from interactions with exogenous sources such as xenobiotic compounds. Oxidative stress occurs when ROS collapse the antioxidant cell defense system, either through increased concentration or due to decreased cell antioxidant capacity. This reaction damages nucleic acids, proteins, and lipids. It is also involved in various pathological processes such as carcinogenesis, neurodegeneration, atherosclerosis, diabetes and aging. In fact, there are a multitude of diseases that have been related to oxidative stress and the generation of free radicals. Therefore, antioxidant therapies and antioxidant-rich or -enriched diets help to prevent, or at least decrease, organic functional deterioration caused by excessive oxidative stress.

Fortunately, the human body has developed a number of enzymatic and non-enzymatic defence mechanisms against the harmful effects of ROS. Thus, the antioxidant enzymes, such as superoxide dismutase (SOD), catalase (CAT) and glutathione peroxidase (GPx), play an important role in the prevention of damage caused by free radicals in living organisms.

Physical exercise, undertaken regularly, has many health benefits, including a decrease in mortality and a lower risk of cardiovascular disease, cancer and diabetes. However, prolonged and intense musculoskeletal contraction generates free radicals and causes oxidative damage to cell components. Accordingly, induction of oxidative stress during physical exercise has been proposed as a cause of damage to the myocyte membrane, leading to an exacerbated inflammatory response and, consequently, to excessive pain and muscle fatigue following exercise.

The World Health Organization defines probiotics as live microorganisms that have beneficial health effects when administered in appropriate amounts. In recent years, there has been a significant increase in the number of studies, both *in vitro* and *in vivo,* related to the antioxidant properties of probiotics (Kleniewska, P., et al., 2016, Oxidative Medicine and Cellular Longevity, doi:10.1155/2016/1340903; Poljsak, B., 2011, Oxidative Medicine and Cellular Longevity, doi:10.1155/2011/194586; Hybertson, B. M., et al., 2011, Mol. Aspects Med., 32, 234-246; Banegas JR, et al., 2006, Rev Esp Cardiol., 6: 3-12).

One example is the study described by Ali Akbar Mohammadi, *et al.* (Ali Akbar Mohammadi, et al., 2015 Int J Prev Med., 6: 82) which analysed and demonstrated the antioxidant and anti-inflammatory effect of a probiotic capsule, administered as a nutritional supplement at 1 capsule/day for 6 weeks to workers in the petrochemical industry. Said capsule consisted of a mixture of the bacterial species *Lactobacillus casei, L. acidophilus, L. rhamnosus, L. bulgaricus, Bifidobacterium breve, B. longum* and *Streptococcus thermophilus.* The results of this study led to the conclusion that the probiotic capsule had beneficial effects on oxidative stress biomarkers.

A particular strain of *L. casei,* described in CN101333505, has also been proposed as an antioxidant product.

Moreover, bacterial strains *B. longum* CECT7347 and *L. casei* CECT9104 have been described in combination with *B. animalis* subsp. *lactis,* as part of a probiotic composition, for use in the treatment and/or prevention of atopic dermatitis (EP3272396).

Another example of probiotic composition comprising a strain of *B. animalis* subsp. *lactis* and the strains *B. longum* CECT7347 and *L. rhamnosus* CECT8361 has been described in file EP32222282, where it is proposed for use in the treatment and / or prevention of psoriasis.

Finally, file EP3241893 describes a formulation comprising a strain of *B. longum* CECT7347 and of *L. rhamnosus* CECT8361 for use in boosting male fertility.

In short, it is desirable to have alternative probiotic compositions to those already existing, with antioxidant properties capable of reducing the harmful effects cuased by oxidative stress at the molecular level in the body, especially after intense physical exercise.

### DESCRIPTION OF THE INVENTION

This invention relates to a probiotic composition consisting of the bacteria *Lactobacillus rhamnosus, Lactobacillus casei* and *Bifidobacterium longum,* preferably of the strains *L*. *rhamnosus* CECT8361, *L. casei* CECT9104 and *B. longum* CECT7347, together with food-based and/or pharmaceutically acceptable vehicles and/or excipients, for use as an antioxidant. This composition is particularly useful in the treatment and / or prevention of damage, at the molecular level, caused by oxidative stress during or after physical exercise.

The strains *L. rhamnosus* CECT8361, *L. casei* CECT9104 and *B. longum* CECT7347, were isolated from the faeces of a Spanish baby under 3 months of age fed exclusively by breastfeeding. In all cases the strains were isolated in selective medium for lactobacilli and bifidobacteria and identified unambiguously by sequencing the 16S rRNA gene.

The examples shown below demonstrate that the intake by humans of the composition of the invention, preferably daily and for six weeks, decreases the oxidative damage to lipids and DNA resulting from high-intensity and long-duration physical exercise. In particular, levels of serum malondialdehyde and oxidized LDL (indicative of oxidative lipid damage) and urinary 8-oxo 2-deoxyguanosine (indicative of oxidative DNA damage) increased less in subjects that consumed the composition of the invention. It is thus shown that intake of the composition of the invention improves the antioxidant status of subjects. Finally, these examples show that the administration of the composition of the invention is safe, given that no adverse effects associated with its intake, nor changes in blood count or liver and kidney function of the study subjects were observed during the clinical trial.

Therefore, one aspect of the invention relates to a composition, hereinafter "composition of the invention", which consists of the bacteria *Lactobacillus rhamnosus, Lactobacillus casei* and *Bifidobacterium longum,* together with one or more vehicles and/or food additives and/or pharmaceutically acceptable vehicles.

The composition of the invention is a probiotic composition. 'Probiotic composition' means a composition comprising at least one living micro-organism or parts thereof which, when ingested, interacts with the individual's metabolism and produces a beneficial effect on the individual.

In a preferred embodiment of the composition of the invention, *L. rhamnosus* is the strain BPL0015 deposited in the Spanish Type Culture Collection under deposit number CECT8361, *L. casei* is strain BPL0004 deposited in the Spanish Type Culture Collection under deposit number CECT9104 and *B. longum* is the strain IATA-ES1 deposited in the Spanish Type Culture Collection under deposit number CECT7347.

*Lactobacillus rhamnosus* is a bacterium found mostly in fermented products (dairy and plant-based), as well as in infant milks. The scientific classification of *L. rhamnosus* is: Kingdom: *Bacteria,* Phylum: *Firmicutes,* Class: *Bacilli,* Order: *Lactobacillales,* Family: *Lactobacillaceae,* Genus: *Lactobacillus,* Species: *Lactobacillus rhamnosus.*

The strain *L. rhamnosus* CECT8361 was isolated from the faeces of a healthy child under three months of age, fed exclusively by breastfeeding. This strain was deposited on 27^{th} May, 2013 under the Budapest Treaty in the Spanish Type Culture Collection, as International Depositary Authority (with headquarters in the Edificio 3 CUE, Parc Científic Universitat de Valencia, C/ Catedrático Agustin Escardino, 9, 46980 Paterna (Valencia), SPAIN). The assigned deposit number is CECT 8361. In this invention, this strain will also be referred to as BPL0015.

*Lactobacillus casei* is a bacterium found mostly in fermented products (dairy and plant-based), as well as in infant milks. The scientific classification of *L. casei* is: Kingdom: *Bacteria,* Phylum: *Firmicutes,* Class: *Bacilli,* Order: *Lactobacillales,* Family: *Lactobacillaceae,* Genus: *Lactobacillus,* Species: *Lactobacillus casei.*

The strain *L. casei* CECT9104 was isolated from the faeces of a healthy child under three months of age, fed exclusively by breastfeeding. This strain was deposited on the 25^{th} February, 2016 under the Budapest Treaty in the Spanish Type Culture Collection, as International depositary Authority (with headquarters in the Edificio 3 CUE, Parc Científic Universitat de Valencia, C/ Catedrático Agustín Escardino, 9, 46980 Paterna (Valencia), SPAIN). The assigned deposit number is CECT9104. This strain will also be referred to as BPL0004 in this invention.

*Bifidobacterium longum* is a Gram-positive, catalase-negative, bifid-shaped bacterium that is commonly found in the gastrointestinal tract, where it produces mainly acetic and lactic acid. The scientific classification of *B. longum* is: Kingdom: *Bacteria,* Phylum: *Firmicutes,* Class: *Actinobacteria,* Order: *Bifidobacteriales,* Family: *Bifidobacteriaceae,* Genus: *Bifidobacterium,* Species: *Bifidobacterium longum.*

The strain *B. longum* CECT 7347 was isolated from the faeces of a healthy infant under three months of age fed exclusively by breastfeeding and deposited on the 20^{th} December, 2007 under the Budapest Treaty at the Spanish Type Culture Collection, International Depositary Authority (with headquarters at the Edificio 3 CUE, Parc Científic Universitat de Valencia, C/ Catedrático Agustín Escardino, 9, 46980 Paterna (Valencia), SPAIN). The assigned Deposit number is CECT7347. This strain will also be referred to as IATA-ES1 or ES1 in this invention.

Another aspect relates to a composition comprising the strain *L. rhamnosus* BPL0015 deposited in the Spanish Type Culture Collection under deposit number CECT8361, the strain of *L. casei* BPL0004 deposited in the Spanish Type Culture Collection under deposit number CECT9104 and strain *B. longum* IATA-ES1 deposited in the Spanish Type Culture Collection under deposit number CECT7347. In a preferred embodiment, this composition also comprises one or more vehicles and/or excipients that are food and / or pharmaceutically acceptable. In another preferred embodiment, this composition also comprises another micro-organism, preferably another bacterium. This other bacterium may belong to the bacterial genera described above *(Bifidobacterium* and *Lactobacillus),* as well as to other bacterial genera such as, but not limited to, *Bacillus, Lactococcus, Pediococcus, Streptococcus* or *Veillonella,* among others; as well as to yeast species belonging to the genera *Kluyveromyces, Pichia* or *Saccharomyces,* among others. In another preferred embodiment, this composition also comprises one or more active substances with antioxidant effect, such as but not limited to fatty acids, glutathione, plant extracts (especially those rich in polyphenols such as anthocyanins, carotenoids, curcumin, lycopene, lutein, melatonin, resveratrol or zeaxanthin), peptides, selenium, vitamins such as A, C or E, among others.

Within this invention are also those bacteria derived from *L. rhamnosus, B. longum* and *L. casei* (or its corresponding strains *L. rhamnosus* BPL0015 CECT8361, *L. casei* BPL0004 CECT9104 and *B. longum* IATA-ES1 CECT7347) and these can form part of the probiotic composition of the invention as an alternative to the specifically indicated strains, provided that they retain the ability to prevent, remit and/or improve damage caused by oxidative stress in the organism. Examples of strains derived from the specifically mentioned strains of the invention may be mutants and genetically modified organisms that present variations in their genome compared to the genome of the strains described in the invention, but where such variations do not affect the ability of the strains to prevent, remit and/or improve damage caused by oxidative stress to the organism. Strains derived from *L. rhamnosus, B. longum* and *L. casei* (or its corresponding strains *L. rhamnosus* BPL0015 CECT8361, *L. casei* BPL0004 CECT9104 and *B. longum* IATA-ES1 CECT7347) may occur naturally or intentionally by mutagenesis methods known in the state-of-the-art such as, for example, but not limited to, the growth of the original strain in the presence of mutagenic or stress-causing agents or by genetic engineering aimed at obtaining the desired mutation. Also contemplated are genetically modified organisms derived from *L. rhamnosus, B. longum* and *L. casei* (or its corresponding strains *L. rhamnosus* BPL0015 CECT8361, *L. casei* BPL0004 CECT9104 and *B. longum* IATA-ES1 CECT7347) which retain the capacity to prevent, remit and/or improve damage caused by oxidative stress in the body and therefore to be used in the treatment and/or prevention of oxidative stress. An assay to check whether a micro-organism has the ability to prevent, remit and/or improve damage caused by oxidative stress in the body is described in the examples accompanying this description.

Furthermore, the present invention also covers cellular components, metabolites and / or molecules secreted by *L. rhamnosus, B. longum* and *L. casei* (or its corresponding strains *L. rhamnosus* BPL0015 CECT8361, *L. casei* BPL0004 CECT9104 and *B. longum* IATA-ES1 CECT7347), as well as compositions that comprise said cellular components, metabolites and/or secreted molecules, and uses thereof for the treatment and/or prevention of oxidative stress. The "cellular components" may include components of the cell wall (such as, for example, peptidoglycan), nucleic acids, membrane components, or others such as proteins, lipids and carbohydrates, and combinations thereof (such as lipoproteins, glycolipids or glycoproteins). The "metabolites" include any molecule produced or modified by the bacterium as a result of its metabolic activity, during its growth, its use in technological processes or during the storage of the product (composition of the invention). Examples of these metabolites include, but are not limited to, organic and inorganic acids, proteins, peptides, amino acids, enzymes, lipids, carbohydrates, lipoproteins, glycolipids, glycoproteins, vitamins, salts, minerals and nucleic acids. "Secreted molecules" include any molecule secreted or released by the bacteria during its growth, its use in technological processes (e.g. food or drug processing) or during product storage (the composition of the invention). Examples of these molecules include, but are not limited to, organic and inorganic acids, proteins, peptides, amino acids, enzymes, lipids, carbohydrates, lipoproteins, glycolipids, glycoproteins, vitamins, minerals, salts and nucleic acids.

The term "excipient" refers to a substance that helps the absorption of any of the components contained in the composition of the invention, i.e., of the strains of the invention, or that stabilizes said components and/or helps the preparation of the composition in that it affords consistency or provides flavours that make it more palatable. Therefore, the excipients could act to bind the components (for example, starches, sugars or cellulose), to sweeten, to colour, to protect the active ingredient (for example, to isolate it from air and/or moisture), to form the content of a pill, a capsule, or any other dosage form or to disintegrate it in order to facilitate dissolution of the components, without excluding other types of excipients not mentioned in this paragraph. Therefore, the term 'excipient' is defined as the material added to the active ingredients to facilitate their preparation and stability, to modify their organoleptic properties and/or to determine the physical-chemical properties of the composition and its bioavailability. The "pharmaceutically acceptable" excipient must not impede the activity of the active components of the composition, i.e., it must be compatible with the viability and functionality of the strains of the invention.

The "vehicle" or "carrier" is, preferably, an inert substance. The functions of the vehicle are to facilitate the incorporation of other components or compounds, to facilitate dosage and/or administration and/or to give consistency and shape to the composition. Therefore, the vehicle is a substance used to dilute any of the components contained in the composition of this invention to a certain volume or weight; or that, even without diluting those components, it is able to facilitate dosage and/or administration and/or give consistency and shape to the composition. When the dosage form is liquid, the vehicle is the diluent. Examples of pharmacologically acceptable vehicles include, but are not limited to, water, saline solutions, alcohol, vegetable oils, polyethylene glycols, gelatin, lactose, starch, amylose, magnesium stearate, talc, surfactants, silicic acid, viscose paraffin, perfumant oil, fatty acid monoglycerides and diglycerides, petroetral fatty acid esters, hydroxymethylcellulose, polyvinylpyrrolidone, and similar.

In addition, the excipient and the vehicle must be a food or be pharmacologically acceptable, i.e., they must have been evaluated and approved so that they do not harm the subject to whom the composition of the invention is administered. In addition, the excipient and / or vehicle may be natural, i.e., they exist in nature, or unnatural, those that, if they are in nature, are not found naturally in combination with the bacteria of invention.

Bacteria *L. rhamnosus, B. longum* and *L. casei,* preferably the strains *L. rhamnosus* BPL0015 CECT8361, *L. casei* BPL0004 CECT9104 and *B. longum* IATA-ES1 CECT7347, must be present in the composition of the invention in a therapeutically effective quantity so that they exert their effect of preventing, remitting and/or improving the damage caused by oxidative stress in the organism.

In the present invention, "therapeutically effective quantity" means the quantity which, when administered to a subject, is sufficient to produce the desired effect. As known by an expert skilled in the art, the therapeutically effective amount may vary depending on, for example, age, body weight, general health status, diet and sex of the subject, as well as on the mode and time of administration, or the rate of excretion, among other factors. Thus, in a more preferred embodiment of the composition of the invention, *L. rhamnosus* is at a concentration of 45%, *L. casei* is at a concentration of 45% and *B. longum* is at a concentration of 10%, compared to the total concentration of bacteria included in the composition.

In another preferred embodiment, the total amount of bacteria in the composition of the invention is 10⁹ CFU.

The composition of the invention may be formulated for pharmaceutical administration, i.e., forming part of pharmaceutical products that will be administered to a subject (for example, orally, topically, etc), and/or for food administration, i.e., forming part of food that is consumed in a subject's diet, and/or it can be administered as a nutritional complement or supplement. Therefore, in another preferred embodiment, the composition of the invention is a pharmaceutical or food composition.

The "pharmaceutical composition" or "drug" is a set of active components or compounds that is made up of, at least, micro-organisms *L. rhamnosus, B. longum* and *L. casei,* preferibly, *L. rhamnosus* BPL0015 CECT8361, *L. casei* BPL0004 CECT9104 and B. *longum* IATA-ES1 CECT7347, at any concentration, preferably those indicated above, and which, in addition, may comprise one or more components or compounds having some biological and/or pharmacological activity which, after administration to a subject, may increase, reinforce and/or boost the activity of the strains included in the composition of the invention. As understood by the expert skilled in the art, the additional components or compounds must be compatible with the bacteria of the composition of the invention. In the context of the present invention, veterinary compositions are also included within the term "pharmaceutical composition".

"Food composition" or "nutritional composition" refers to a food or nutritional supplement that beneficially affects one or more bodily functions, thus improving the state of health and well-being of the individual who consumes it. In this invention, said food composition is intended to prevent, remit and/or improve damage caused by oxidative stress to the body. The food composition referred to in this invention includes, but is not limited to, a food, a functional food, a probiotic, or nutritional complement or supplement. In cases where the composition of the invention is formulated as a nutritional composition, said composition may be a food or be incorporated into a food or foodstuff intended for animals, preferably humans. Thus, in a more preferred embodiment, the food composition is selected from a foodstuff (which can be, but is not limited to, a food for specific nutritional purposes or a medicinal food) and a nutritional supplement.

The term "supplement" or "additive", are synonymous with any of the following terms; "dietary supplement", "nutritional supplement", "food supplement", "diet supplement" or "dietary additive", and similar, and refer to products or preparations intended to supplement the normal diet, consisting of concentrated sources of nutrients or other substances having a beneficial nutritional or physiological effect on the individual. In the present invention, the "substance" that has a beneficial nutritional or physiological effect on the individual consists of the micro-organisms *L. rhamnosus, B. longum* and *L. casei,* preferibly, *L. rhamnosus* BPL0015 CECT8361, *L. casei* BPL0004 CECT9104 and B. *longum* IATA-ES1 CECT7347, which form part of the composition of the invention. The food supplement can be found in a simple or combined form and marketed in dosage form, i.e., in capsules, tablets, pills and other similar forms, sachets of powder, ampoules of liquid and dropper bottles and other similar forms of liquids and powders to be taken in a unit amount.

The composition of the invention may also be part of the so-called "special group nutritional foods or supplements", i.e. foods or supplements that meet particular nutritional needs. In particular, the composition of the invention is preferably intended for individuals who perform intense physical exercise, more preferably on a regular basis.

Examples of foods that may comprise the composition of the invention include, but are not limited to, feed, dairy products, plant products, meat products, snacks, chocolates, beverages, dehydrated powdered foods, food gels, baby foods, cereals, fried foods, industrial pastry and cookies. Examples of dairy products include, but are not limited to, products derived from fermented milk (e.g., yogurt or cheese) or unfermented milk (e.g., ice cream, butter, margarine or whey). The plant-based product is, for example, a cereal in any form, fermented (e.g. a fermented soy-based product or fermented oat-based product) or unfermented, and an aperitif. Beverages may be unfermented milk or smoothies in liquid form or in powder for reconstitution with water. In a particular embodiment, the feed or food product comprising the composition of the invention is selected from the group consisting of: fruit or vegetable juices, ice-cream, infant formula, milk, yogurt, cheese, fermented milk, powdered milk, cereals, pastry products, dairy products, meat products, beverages and confectionery, gum-based products (for example, fruit gums, with or without added sugars).

In another preferred embodiment, the composition of the invention is formulated for oral administration.

In another preferred embodiment, the composition of the invention is administered to an individual through diet.

The dosage form of the composition of the invention shall be adapted to the route of administration used. Therefore, the composition may be formulated as a solution, suspension, emulsion, syrup or any other clinically permitted dosage form. Taking into account that the preferred route of administration is oral, the composition of the invention is preferably presented in solid, semi-solid or liquid form, more preferably solid, for oral administration. Examples of solid formulations include tablets, capsules, powders, granules or granulated products, particles or coated tablets, suppositories, tablets, pills, gels, dispersible films or microspheres. More preferably, the composition of the invention is presented in capsule form.

Alternatively, sustained-release forms can be used to deliver the composition of the invention, including, for example, its encapsulation in liposomes, micro-bubbles, microparticles or microcapsules, and similar. Appropriate sustained-release forms, as well as materials and methods for their preparation, are widely known in the state-of-the-art. Thus, the orally administered form of the composition of the invention could be a sustained-release form that additionally comprises a coating or matrix. The sustained-release coating or matrix includes, but is not limited to, water-insoluble or modified, natural, semi-synthetic or synthetic polymers, proteins, waxes, fats, fatty alcohols, fatty acids, semi-synthetic or synthetic natural plasticizers, or a combination of two or more of the above. Enteric coatings can be applied using conventional processes known to experts in the art.

Another aspect of the invention relates to the composition of the invention for use as a medicinal product.

The term "medicine", as used in this specification, refers to any substance used for the prevention, relief, treatment, reduction or cure of diseases or clinical conditions in animals, preferably in humans. In the context of this invention, the disease or clinical condition is oxidative stress or molecular damage to the body, preferably to lipids and DNA, caused by oxidative stress.

Another aspect of the invention relates to the composition of the invention for use in the treatment and/or prevention of oxidative stress in an individual, or in the treatment and/or prevention of molecular damage, preferably to lipids and DNA, caused by oxidative stress in an individual. Preferably, oxidative stress is caused by physical activity or physical exercise.

"Oxidative stress" is a condition caused by an imbalance between the production of reactive oxygen species and/or peroxides and the body's ability to repair the resulting damage. Imbalances in this normal redox state of cells can cause toxic effects through the production of peroxides and free radicals that damage all cell components, including proteins, lipids, and DNA. In humans, oxidative stress, and hence the so-called reactive oxygen species (ROS), are involved in the main aetiopathogenic mechanisms, or their consequences, in more than one hundred diseases of great clinical and societal importance, such as atherosclerosis, Parkinson's disease, myalgic encephalopathy, multiple chemical sensitivity, periodontitis, varicocele and Alzheimer's disease and may also be important in aging.

Oxidative stress is caused by an imbalance between the production of reactive oxygen species and the ability of the biological system to restore intermediate reagents and/or repair the resulting damage. The effects of oxidative stress depend on the magnitude of the changes that have occurred, and whether the cell is able to overcome the small disturbances and regain its original state. Moderate oxidation can trigger apoptosis, while severe oxidative stress can cause necrosis and even cell death. One particularly destructive aspect of oxidative stress is the production of ROS, which include free radicals and peroxides. Most of these ROS are produced at a low level under normal aerobic metabolic conditions and the cell damage they cause is constantly repaired. However, under the severe levels of oxidative stress caused by necrosis, the damage results in ATP depletion preventing cell death by controlled apoptosis, causing the cell to die by releasing numerous cytotoxic compounds into the medium.

As used in the present invention the term "physical exercise" or "physical activity" refers to any bodily movement produced by skeletal muscles that requires energy expenditure and which also consists of planned, structured, repetitive physical activity, performed with a goal related to improvement or maintenance of one or more components of physical fitness, for example, sport. This term includes professional as well as recreational or leisure physical exercise. Furthermore, day-to-day tasks (work, home care and maintenance, care of the family, etc.) often involve energy expenditure comparable to that spent after directed physical exercise. Therefore, such duties are also included within the term "physical activity" as used in this invention. The term includes intense physical wear caused by high-intensity daily chores (work with high physical wear, home and family care, etc.). The physical exercise or physical activity referred to in this invention may be aerobic or anaerobic, preferably aerobic.

Physical exercise or physical activity referred to in the present invention is intense physical exercise, i.e., of high intensity and long duration (preferably of at least 30 minutes, more preferably of at least 60 minutes; or > 6 MET). The intensity reflects the speed at which the activity is carried out, or the magnitude of the effort required to perform such an exercise or activity. The intensity of different forms of physical activity varies from person to person. The intensity of physical activity depends on how much each person exercises and their physical fitness. METs are used to express physical activity. METs is the ratio between the ratio of a person's working metabolic rate relative to their resting metabolic rate (1 MET = the energy cost of sitting quietly and is equivalent to a consumption of 1 kcal/kg/h). Compared to this situation, caloric consumption is estimated to be about 3 to 6 times higher (3-6 METs) when moderate intensity activity is performed and more than 6 times higher (> 6 METs) when vigorous activity is performed.

The composition of the invention can be administered before, during or after physical exercise, preferably before or during, more preferably before.

Most preferably, the composition of the invention is administered once a day, preferably at breakfast, and even more preferably for 6 weeks.

In another preferred embodiment, the composition of the invention is for use in the treatment and/or prevention of diseases or clinical conditions related to or associated with oxidative stress, such as, for example, but not limited to, cancer, neurodegenerative diseases, atherosclerosis and diabetes.

"Prevention" means preventing molecular damage, preferably to lipids and DNA, associated with oxidative stress in an individual, in particular when the individual is predisposed to it, for example, because he or she usually performs intense physical exercise.

The term "treat" or "treatment" comprises inhibiting or remitting molecular damage, preferably to lipids and DNA, associated with oxidative stress.

Another aspect of the invention relates to the use of the composition of the invention as an antioxidant. This use refers, preferably, to a non-therapeutic use, i.e., a cosmetic use, more preferably for the treatment and/or prevention of aging in a subject.

The term "subject", "individual" or "organism", as used in the present invention, refers to any animal, preferably healthy, belonging to any species, preferably mammals, more preferably humans. Examples of subjects include, but are not limited to, animals of commercial interest such as poultry (hens, ostriches, chickens, geese, partridges, etc.), rabbits, hares, domestic animals (dogs, cats, etc.), ovine and caprine livestock (sheep, goats, etc.), porcine livestock (wild boars, pigs, etc.), equine livestock (horses, ponies, etc.), cattle or bovine livestock (bulls, cows, oxen, etc.), game or quarry, such as deer, reindeer, etc., and humans. In a particular embodiment, the subject is a mammal, preferably a human being of any race, sex or age.

Throughout the description and claims the word "comprises" and its variants are not intended to exclude other technical characteristics, additives, components or steps. The following examples and figures are provided as way of example, and are not intended to limit this invention.

### DESCRIPTION OF THE FIGURES

**Fig. 1****. Serum malondialdehyde for each of the groups (placebo and probiotic)**, for each test and in the initial and final stages of each test. * p<0.05 comparison between the initial and final stage for each test.
**Fig. 2****. Increase in serum malondialdehyde during physical exercise tests for each group (placebo and probiotic).** * p<0.05 Test comparison.
**Fig. 3****. Serum oxidised LDL for each of the groups (placebo and probiotic)**, for each test and at the initial and final stages of each test. * p<0.05 comparison between the initial and final stage for each test.
**Fig. 4****. Increase in serum oxidised-LDL during physical exercise tests for each group (placebo and probiotic).** * p<0.05 Test comparison.
**Fig. 5****. 8-oxo 2' - deoxiguanosin in 24-hour urine (pg/ml) for each of the groups (placebo and probiotic)**, for each test and at the initial and final stages of each test. * p<0.05 comparison between the initial and final stage for each test.
**Fig. 6****. Increase in 8-oxo 2' - deoxiguanosin in urine for 24 hours (pg/ml) during physical exercise tests for each of the groups (placebo and probiotic).** * p<0.05 Test comparison.

### EXAMPLES

Below, we will show the invention using a nutrition clinical trial conducted by the inventors, which highlights the effectiveness of the composition of the invention in reducing molecular damage caused by oxidative stress during high-intensity long-duration physical exercise.
Example 1. Clinical trial to determine the effectiveness of the composition of the invention, compared to a placebo, in the reduction of oxidative stress during high-intensity long-duration physical exercise, as well as to determine the tolerance and safety of the composition.

### 1.1. Study design

A randomized, placebo-controlled clinical trial was conducted with two parallel study groups based on the product consumed (experimental or placebo), double-blind and unicentric, designed to evaluate the effect of the product on the reduction of oxidative stress produced by high-intensity long-duration physical exercise.

The selected subjects were healthy caucasian male subjects aged 18 to 45, selected from the general population, performing aerobic physical exercise 2 to 4 times a week. Excluded from the study were those subjects with a history of any chronic disease, especially digestive tract diseases, who had undergone abdominal surgery in the three months prior to the study, with a history of bronchial asthma or chronic obstructive pulmonary disease, reactive respiratory tract disease such as bronchial asthma, sinus bradycardia, second or third degree atrioventricular block, manifest heart failure or cardiogenic shock, a history of allergic hypersensitivity or poor tolerance to any component of the products under study, participation in another clinical trial in the three months prior to the study, subjects diagnosed and/or being treated for high blood pressure, smokers (>10 cigarettes a day), subjects with body mass index greater than 35 kg/m2 (BMI>30), subjects with a history of drug or alcohol abuse or of other substances or other factors limiting their ability to cooperate during the study.

As a control, a placebo with identical organoleptic characteristics and the same visual aspect as the trial product was selected.

The characteristics of the study product were the following:
- Pharmaceutical form: capsules, both the trial product and the placebo.
- Content: in no case did the excipients modify the pharmacokinetics or pharmacodynamics of the active substances, they were added for technological reasons only.
- Route of administration: oral
- Posology: 1 capsule / day.
- Dose regimen: 6 weeks.
The composition of the invention consisted of a mixture of:
- *L. rhamnosus* BPL0015 (CECT8361) (45%)
- *L. casei* BPL0004 (CECT9104) (45%), and
- *B. longum* IATA-ES1 (CECT7347) (10%)

The final product contained 10⁹ CFU / capsule.

### 1.2. Study protocol

In order to demonstrate the proposed objectives, study subjects were subjected to an oxidative stress model consisting of the performance of high-intensity long-duration physical activity (90 minutes). This increased the oxidative stress of the study subjects and showed the efficacy of the product (composition of the invention) compared to the placebo in terms of improving the oxidative status of the subjects. The proposed oxidative model consisted of a preliminary test and two non-maximal stress tests of high and constant intensity, which will henceforth be called test 1 and test 2. Each one is described below.
- Preliminary test: the aim of this test was to be able to calculate individually the intensity at which the subjects (cyclists) should perform the subsequent physical activity, i.e., tests 1 and 2. During the realization of this test the subjects did not ingest either of the products used in the study (probiotic or placebo). This test was carried out on a bicycle roller with electromagnetic resistance (Technogym Spin Trainer) on which the bicycle is placed with a starting load that simulates a speed of 12 km/h with a load increase of 2 Km/h each minute, maintaining a constant slope of 2%. Cyclists employed a free style. In order to calculate the intensity of subsequent physical activity, subjects underwent ergospirometric and electrocardiographic monitoring. Thus, previously, the subject was prepared for respiratory gases analysis (breath-by-breath, open circuit, gas analyser brand Jaeger Oxicom Pro) while undertaking the test. The main variable evaluated during the performance of this test was the maximum/peak consumption of absolute and relative oxygen (VO₂ max) which is the maximum volume of oxygen measured in ml/min or ml/Kg x min detected in the test or maximum value of that variable after which it does not increase, even if effort intensity is increased. Then, after a 7-day wash-out period of the oxidative stress generated in the preliminary test, the following test was performed.
- 1st stress test (test 1): one week after the first test, the study subjects performed the following, which consisted of high-intensity physical activity of 90 minutes duration. The study subject performed a constant intensity stress test on a bicycle roller with electromagnetic resistance, on which the subject's bicycle was placed. The maximum load maintained was equivalent to a heart rate corresponding to 75% of the subject's maximum oxygen consumption calculated in the preliminary test and a constant slope of 2% was maintained. The aim of this test was to cause high oxidative stress in the subject, so that the antioxidant effect of the trial product and placebo could be evaluated. During the test, the study subjects did not consume any of the products, only water *ad libitum.* Once the subjects had performed test 1, the 6-week period of product consumption (probiotic or placebo) began.
- 2nd stress test (test 2): after the 6-week product consumption period, the study subjects performed test 2, which consisted of the same high intensity physical activity performed in test 1.

Before and after tests 1 and 2, study subjects underwent blood extraction and a 24-hour urine collection. Blood samples were taken half an hour before the subjects performed test 1 and test 2 and half an hour after each test. Likewise, the day before and after each test, 24-hour urine collection was performed. After measuring the total volume of urine excreted within 24 hours, a sample of 9 ml was extracted and frozen at -80 ° C in three different cryovials until further analysis.

### 1.3. Study variables analyzed

All variables were analysed at baseline and after 6 weeks of uninterrupted consumption of the product.

### 1.3.1. Variables of oxidative damage caused by high-intensity long-duration physical exercise.

Both aerobic and anaerobic physical exercise lead to an increase in the production of free radicals. Certain levels of these oxidizing compounds have positive effects on the body's immune functions, tissue replacement and cell resistance, and even on muscle contraction and adaptation to systematic exercise. However, physical exercise can trigger an imbalance between free radical production and antioxidant defense mechanisms in the organism, leading to different types of molecular damage, evidenced via different biological markers of molecular damage on lipids, proteins and DNA. In this study, the subjects were subjected to a source of oxidative stress (test 1 and test 2) in order to evaluate the antioxidant effect of the probiotic versus a placebo, i.e., the ability to slow down oxidative damage caused by high-intensity long-duration physical exercise that can exceed antioxidant defence mechanisms.

### Oxidative damage to lipids

- Serum malondialdehyde analysis. Serum malondialdehyde was analyzed with the MDA oxLDL ELISA (MDA (Malondialdehyde) ELISA KIT ELABSCIENCE Houston, Texas (USA)). This analysis was made of the serum obtained from the blood extractions performed half an hour before and after each of the stress tests performed.
- Oxidized LDL analysis. Serum LDL in its oxidized form was quantified using the MDA oxLDL ELISA (Human OxLDL (Oxidized Low Density Lipoprotein) ELISA KIT ELABSCIENCE Houston, Texas (USA)). This analysis was made of the serum obtained from the blood extractions performed half an hour before and after each of the stress tests performed.

### Oxidative damage to DNA

Analysis of 8-oxo 2-deoxiguanosin in 24-hour urine. The 8-oxo-2-deoxiguanosin in 24-hour urine was analyzed using the DNA/RNA Oxidative Damage EIA Kit (80HdG (8-Hydroxideoxyguanosine) ELISA KIT ELABSCIENCE Houston, Texas (USA)). This analysis was made in 24-hour urine samples collected before and after each stress test.

### 1.3.2. Safety variables.

Biochemical blood profile was analyzed to determine GOT, GPT, GGT, LDH enzyme values, and of bilirubin to assess liver function, and of biomolecules such as urea and creatinine to evaluate renal function. A blood count was also performed to evaluate the red, white and platelet cells. Blood samples were obtained twice during the study, at baseline and the end.

Adverse events were also registered and evaluated.

### 1.4. Statistical analysis

A descriptive analysis (mean and standard deviation) was made of all the variables under study, both at baseline for each of them and their development. This analysis was conducted for the total group of subjects participating in the study.

The homogeneity of the population at baseline with respect to demographic variables, medical history and other clinical parameters was also analyzed. For quantitative variables, comparisons of t-Student were made between the two study groups. The qualitative variables were analyzed by means of a homogeneity test based on Chisquare distribution when made possible by the expected values and otherwise by means of an exact Fisher test.

In order to analyse the inter-group differences (experimental and control) for the trends in the different variables, an analysis of variance was performed for repeated measurements with two intra-target factors (test: before consumption and after 8 weeks of consumption and time: before and after each test) and an inter-target factor (product: experimental product and placebo product). In this way, differences were established in each of the variables analysed, taking into account these factors. Tukey or Bonferroni tests were run for the post-hoc analysis. Comparisons were made for those significant effects with the option of assuming or not equal variances.

In the set of statistical tests the level of significance used was 0.05. The statistical analysis was carried out with SPSS 21.0. software.

### 1.5. Results

The study was started by 45 subjects, one of whom was excluded before the first test. The remaining 44 subjects were randomized into the two study groups. During the study, one subject in the placebo group was withdrawn for not attending follow-up visits. Therefore, 43 subjects were analyzed: 22 who consumed the probiotic product and 21 who consumed the placebo product.

In the group that consumed the probiotic product, the average age was 25.3 ± 7.2 years, while in the placebo group, the average age was 27.1 ± 8.4 years.

### 1.5.1. Serum malondialdehyde analysis.

Descriptive statistics are presented in the following table:

**Table 1. Statistical levels of malondialdehyde in serum (ng/ml) (mean, standard error, mean difference, P1 level of statistical significance for the difference between levels before and after each test and P2 level of significance for the difference in the increase of the levels for the placebo and probiotic).**

| | | | Mean | S. E. | Mean Dif. | P1 | P2 |
|---|---|---|---|---|---|---|---|
| **Placebo** | Test 1 | Before | 347.4 | 84.8 | 143.7 | .094 | .975 |
| | | Afterwards | 491.1 | 145.3 | | | |
| | Test 2 | Before | 312.9 | 64.3 | 141.5 | .149 | |
| | | Afterwards | 454.4 | 113.3 | | | |
| **Probiotic** | Test 1 | Before | 433.2 | 82.9 | 254.2 | .003 | .005 |
| | | Afterwards | 687.4 | 142.0 | | | |
| | Test 2 | Before | 358.0 | 62.9 | 46.6 | .623 | |
| | | Afterwards | 404.6 | 110.7 | | | |

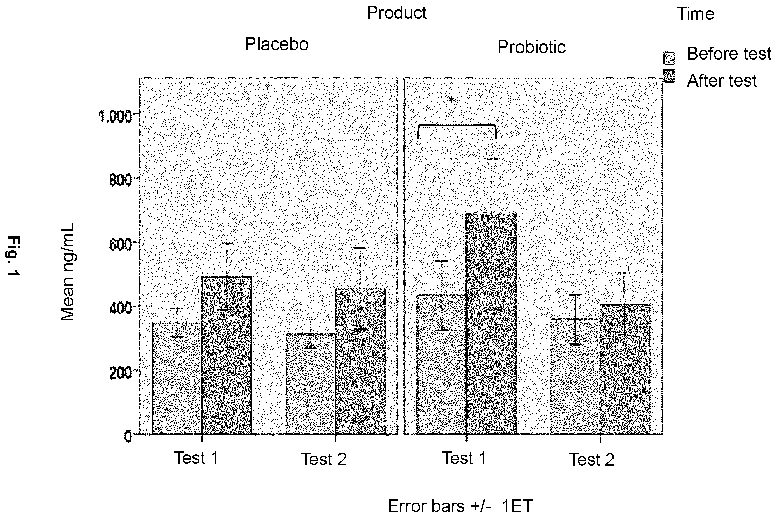

The following was obtained in the comparative analysis:
- Comparison of the values of the variable in the initial state. There are no significant differences when comparing the values of this variable at the initial stage, so it can be said that the groups were homogeneous for this variable at the initial stage of each test.
- Placebo group. During the first test results show a non-significant increase (P<0.094) in serum malondialdehyde levels, secondary to the damage generated by high-intensity long-duration physical exercise. On performing the second test, after ingestion of the placebo product, physical exercise produced the same increase in the levels of this parameter as in test 1 (p<0.149). Therefore, one cannot affirm that the consumption of the placebo altered the trends in this variable during the realization of the stress tests (Fig. 1).
- Experimental group. There is a significant increase (P<0.001) in serum malondialdehyde levels during the first test. On performing the second test, following the intake of the probiotic product, physical exercise resulted in a much lower increase in this parameter than in test 1 (p<0.623). When comparing the changes in this parameter in test 1 with those obtained in test 2, significant differences were observed (p<0.005), i.e., in the second test, the subjects who consumed the probiotic product showed a lower increase in malondialdehyde than during the first test (Fig. 1). Therefore, one can state that the consumption of the probiotic product changed the trends in this variable during the stress tests.

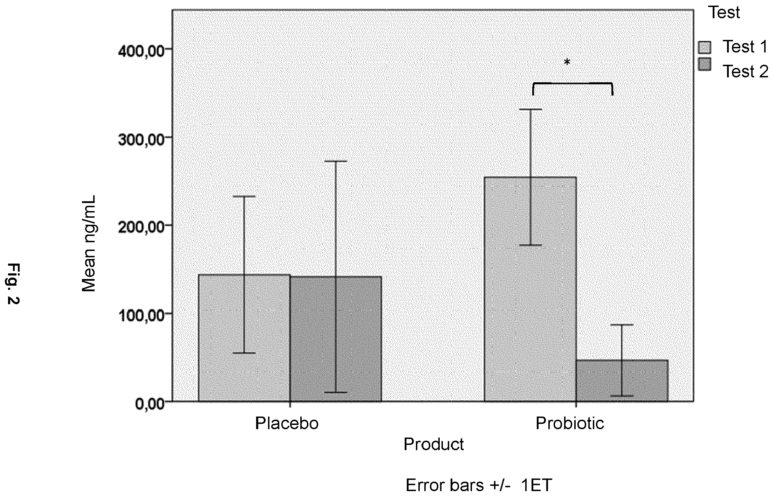

On comparing the trends between the two groups (Fig. 2), significant differences (p=0.047) were observed, i.e., one can state that the trends for both products differ, so it can be concluded that the 6-week intake of the probiotic product produces significant improvements compared to the placebo for this variable.

### 1.5.2. - Oxidized LDL analysis.

Descriptive statistics are presented in the following table:

**Table 2. Statistical levels of oxidized LDL in serum (ng/ml) (mean, standard error, mean difference, P1-level of statistical significance for the difference between the levels before and after each test and P2 level of significance for the difference in the increase of the levels for the placebo and probiotic).**

| | | | Mean | S.E. | Mean dif. | P1 | P2 |
|---|---|---|---|---|---|---|---|
| **Placebo** | Test 1 | Before | 740.3 | 61.5 | 159.3 | .000 | .536 |
| | | Afterwards | 899.6 | 64.1 | | | |
| | Test 2 | Before | 777.8 | 69.2 | 196.6 | .001 | |
| | | Afterwards | 974.4 | 78.9 | | | |
| **Probiotic** | Test 1 | Before | 646.2 | 60.1 | 162.8 | .000 | .042 |
| | | Afterwards | 809.0 | 62.6 | | | |
| | Test 2 | Before | 772.9 | 67.6 | 40.4 | .467 | |
| | | Afterwards | 813.3 | 77.1 | | | |

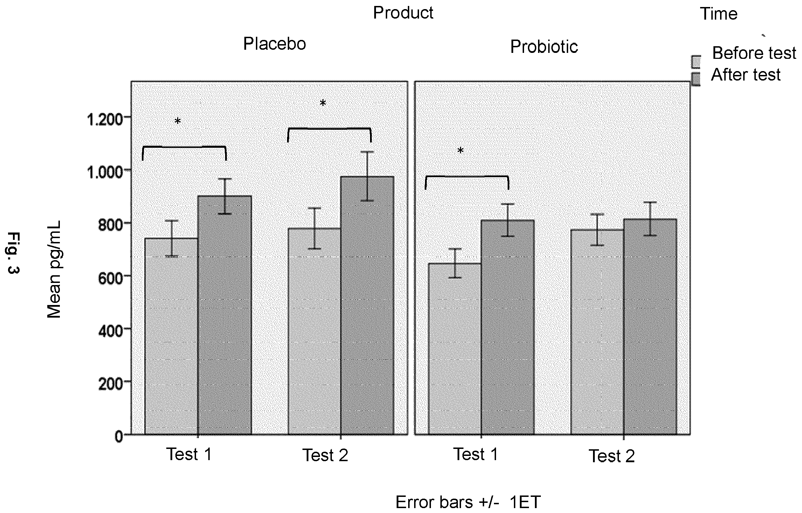

The following was obtained in the comparative analysis:
- Comparison of the values of the variable in the initial state. No significant differences were observed when comparing the values of this variable at the initial stage, so it can be said that the groups were homogeneous for this variable at the initial stage of each test.
- Placebo group. During the first test, a significant increase (P<0.001) in serum oxidized LDL levels secondary to the damage caused by high-intensity long-duration physical exercise was observed. On performing the second test, after ingestion of the placebo product, physical exercise produced the same increase in the levels of this parameter as in test 1 (p<0.001) (Fig. 3). Therefore, it cannot be said that placebo consumption changed the trends in this variable during stress tests.
- Experimental group. A significant increase (P<0.001) in serum oxidised LDL levels was observed during the first test. On performing the second test, following the intake of the probiotic product, physical exercise produced a much lower increase in this parameter than in test 1 (p<0.467) (Fig. 3). Comparison of the changes in this parameter in test 1 with those obtained in test 2 showed significant differences (p<0.042), i.e., in the second test, the subjects who consumed the probiotic product showed a lower increase in oxidized LDL than during the first test. Therefore, one can state that the consumption of the probiotic product changed the trends in this variable during the physical tests.

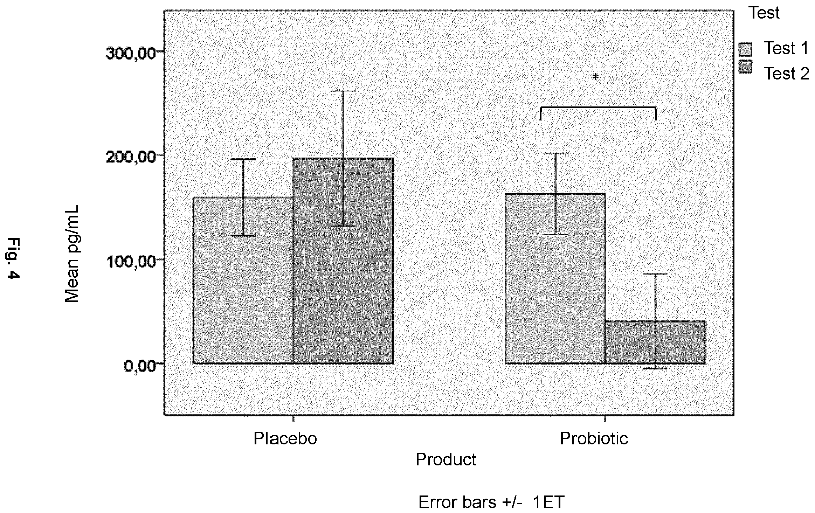

On comparing the trends between the two groups (Fig. 4), significant differences (p=0.05) were observed, i.e., the trends for both products can be said to differ, so it can be concluded that the 6-week intake of the probiotic product produces significant improvements compared to the placebo for this variable.

### 1.5.3. Analysis of 8-oxo 2-deoxiguanosin in 24-hour urine.

Descriptive statistics are presented in the following table:

**Table 3. Statistical levels of 8-oxo 2'-deoxiguanosin in 24-hour urine (pg/ml) (mean, standard error, mean difference, P1-level of statistical significance for the difference between the levels before and after each test and P2 level of significance for the difference in the increase in levels for the placebo and probiotic).**

| | | | | | | | |
|---|---|---|---|---|---|---|---|
| | | | Mean | S.E. | Mean dif. | P1 | P2 |
| **Placebo** | Test 1 | Before | 10.7 | 2.0 | 12.4 | .000 | .620 |
| | | Afterwards | 23.1 | 3.8 | | | |
| | Test 2 | Before | 11.8 | 2.4 | 11.5 | .000 | |
| | | Afterwards | 23,4 | 3,3 | | | |
| **Probiotic** | Test 1 | Before | 13.3 | 2.0 | 15.7 | .000 | .000 |
| | | Afterwards | 29.0 | 3.7 | | | |
| | Test 2 | Before | 13.6 | 2.4 | 4.8 | .007 | |
| | | Afterwards | 18.4 | 3.2 | | | |

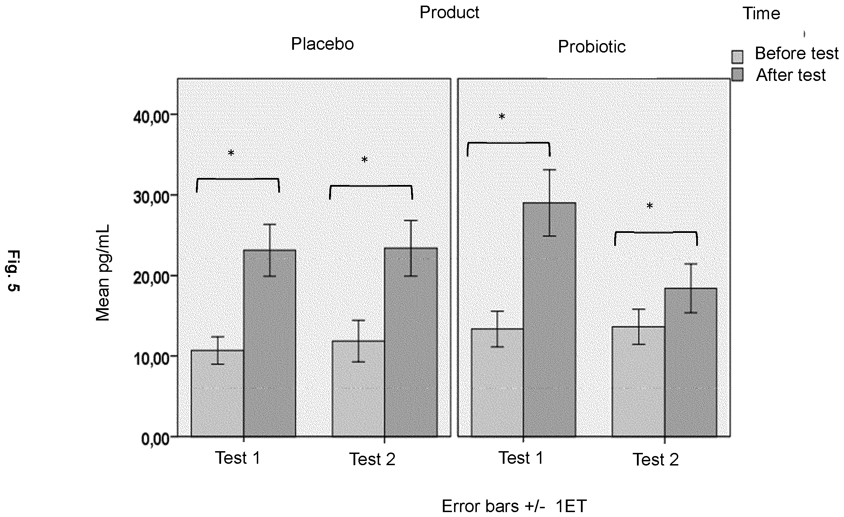

The following was obtained in the comparative analysis:
- Comparison of the values of the variable in the initial state. No significant differences were observed when comparing the values of this variable at the initial stage, so it can be said that the groups were homogeneous for this variable at the initial stage of each test.
- Placebo group. During the first test, a significant increase (p<0.001) in the levels of 8-oxo 2'-deoxiguanosin in 24-hour urine was observed secondary to the damage caused by high-intensity long-duration physical exercise. On performing the second test, after ingestion of the placebo product, physical exercise produced the same increase in the levels of this parameter as in test 1 (p<0.001) (Fig. 5). Therefore, it cannot be said that placebo consumption changed the trends in this variable during stress tests.
- Experimental group. A significant increase (15.7 pg/ml; p<0.001) in 24-hour urine 8-oxo 2'-deoxiguanosin levels was observed during the first test. On performing the second test, following the intake of the probiotic product, physical exercise resulted in a much lower increase in this parameter than in test 1, but was also significant (4.8 pg / ml; p<0.007) (Fig. 5). Comparison of the changes in this parameter in test 1 with those obtained in test 2 revealed significant differences (p<0.001), i.e. in the second test the subjects who consumed the probiotic product had a lower increase of 24-hour urine 8-oxo 2'-deoxiguanosin levels than during the first test (Fig. 5). Therefore, we can state that the consumption of the probiotic product changed the trends in this variable during the stress tests.

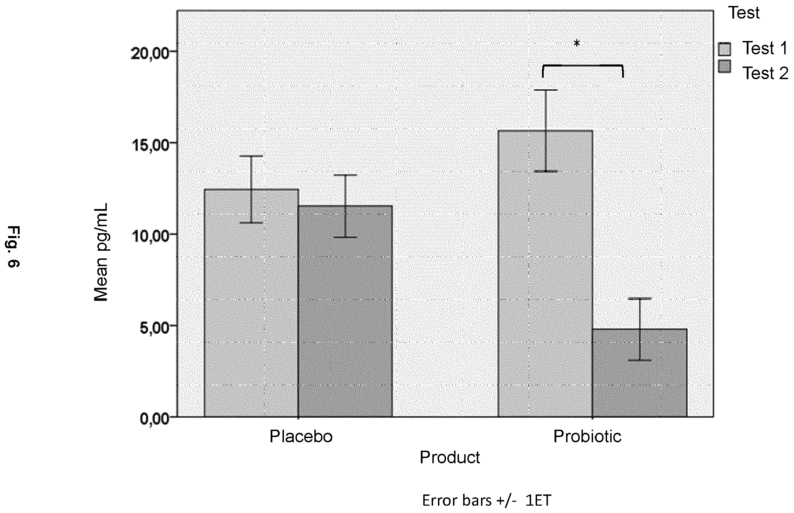

On comparing the trends between the two groups (Fig. 6), significant differences (p=0.001) were observed, i.e., the trends for both products can be said to differ, so it can be concluded that the 6-week intake of the probiotic product produces significant improvements compared to placebo for this variable.

In conclusion, the intake of the probiotic product of the invention for 6 weeks decreased the oxidative damage to lipids and DNA generated by high-intensity long-duration physical exercise.

High-intensity long-duration physical exercise generates oxidative damage to lipids, proteins and DNA. This can be demonstrated by analyzing different metabolites: oxidative damage to lipids causes an increase in serum malondialdehyde and an increase in serum oxidised LDL-cholesterol, and oxidative damage to DNA causes an increase in urine levels of 8-oxo 2'-deoxiguanosin. The intake of probiotic decreased the increase in serum malondialdehyde, serum oxidized chol-LDL and 8-oxo 2'-deoxiguanosin in 24-hour urine. That is to say, the results demonstrate that after ingestion of the probiotic there is a decrease in the oxidative damage to lipids and DNA caused by high-intensity long-duration physical exercise; this exercise has previously been shown to generate oxidative damage to lipids and DNA.

### 1.5.4. Safety variables.

No adverse events related to the intake of the probiotic of the invention were observed in any of the study subjects. No changes were found in the blood count, liver or kidney function of the subjects evaluated. Therefore, the intake of the composition of the invention is safe.

In conclusion, the daily consumption of the probiotic of invention for 6 weeks:
- Decreased oxidative damage to lipids and DNA generated by high-intensity long-duration physical exercise.
- Improved the antioxidant status of subjects.
- No adverse events were observed related to its intake in any of the study subjects, no changes in liver or kidney function in the subjects evaluated, therefore it can be concluded that it is safe.

## Claims

1. A composition that consists of the bacteria *Lactobacillus rhamnosus, Lactobacillus casei* and *Bifidobacterium longum,* together with one or more vehicles and/or excipients, acceptable pharmaceutically and/or as food additives.

2. The composition according to claim 1, where *L. rhamnosus* is the strain BPL0015 deposited in the Spanish Type Culture Collection under deposit number CECT8361, *L*. *casei* is the strain BPL0004 deposited in the Spanish Type Culture Collection under deposit number CECT9104 and *B. longum* is the strain IATA-ES1 deposited in the Spanish Type Culture Collection under deposit number CECT7347.

3. The composition according to either of the claims 1 or 2, where *L. rhamnosus* is at a concentration of 45%, *L. casei* is at a concentration of 45% and *B. longum* is at a concentration of 10%, compared to the total concentration of bacteria included in the composition.

4. The composition according to any of the claims 1 to 3, where the total amount of bacteria in said composition is 10⁹ CFU.

5. The composition according to any of the claims 1 to 4, where said composition is a pharmaceutical or food composition.

6. The composition according to any of the claims 1 to 5, formulated for oral administration.

7. The composition according to any of the claims 1 to 6, where said composition is presented in solid form.

8. The composition according to claim 7, where said composition is presented in capsule form.

9. Composition according to any of the claims 1 to 8, for use as a medicinal product.

10. Composition according to any of the claims 1 to 8, for use in the treatment and / or prevention of oxidative stress in a subject.

11. Composition for use according to claim 10, where oxidative stress is caused by physical activity.

12. Composition for use according to any of the claims 9 to 11, where said composition is administered once a day.

13. Composition for use according to any of the claims 9 to 12, where said composition is administered for 6 weeks.
